# EUROPEAN PATENT APPLICATION

(11) **EP 4 023 153 A1**
(43) Date of publication of application: **06.07.2022**
(21) Application number: 20217967.7
(22) Date of filing: 31.12.2020
(51) Int. Cl.: A61B 5/06, A61B 5/367, A61B 5/00

(54) **QUALITY MEASURE FOR A MAPPING FUNCTION**

(71) Applicant: Koninklijke Philips N.V., 5656 AG Eindhoven (NL)
(72) Inventor: WILDEBOER, Rogier Rudolf, 5656 AE Eindhoven (NL); KAHLMAN, Josephus Arnoldus Johannes Maria, 5656 AE Eindhoven (NL); HENDRIKS, Bernardus Hendrikus Wilhelmus, 5656 AE Eindhoven (NL); SCHÄFER, Dirk, 5656 AE Eindhoven (NL)
(74) Representative: Philips Intellectual Property & Standards

(57) **Abstract**

A mechanism for generating and providing one or more quality measures of a mapping function (for mapping electrical responses of an electrode to a predicted position of that electrode within an anatomical cavity) to a user, such as a clinician. Each quality measure predicts or indicates a quality of a mapping function with respect to a particular part of an anatomical cavity, for instance, indicating a predicted accuracy of the mapping function for predicting a position of an electrode located within a particular portion of the anatomical cavity. A user-perceptible output is provided that indicates the quality measure for the user.

## Description

### FIELD OF THE INVENTION

The present invention relates to the field of medical devices, and in particular to medical devices used for tracking the position of an interventional device within a subject.

### BACKGROUND OF THE INVENTION

Medical imaging is an important aspect in diagnosing and/or treating a subject. There is an increasing interest in the use of dielectric imaging, sometimes referred to as electroanatomical mapping, processes for imaging or modelling of an anatomical cavity and/or structure. Dielectric imaging processes facilitate high quality imaging or modelling of a subject's anatomy without the need for contrast agents and/or X-ray.

In a dielectric imaging process, two or more crossing electrical fields are induced by an array of electrodes positioned on the outside of the subject ("external electrodes"), such as on the surface of the subject. These electric fields induce position dependent electromagnetic responses, such as a voltage response, in electrodes placed within the body (an "internal electrode"). A mapping function is used to predict or "map" the position dependent electromagnetic response to a position in space. The position of an internal electrode can thereby be tracked by monitoring the response of the electrode to these electric fields.

By tracking and iteratively recording the position of the internal electrode(s), a model of the internal anatomy of the subject can be (re)constructed. This is because it can be assumed that the internal electrodes will only be located within cavities of the subject, thereby allowing the bounds of the cavity to be constructed.

One example of a suitable mechanism for constructing a model of the internal anatomy of the subject based on the response of internal electrodes to crossing electric fields induced by external electrodes is disclosed by the European Patent Application having the publication number EP 3568068 A1.

Of course, the mapping function used in a dielectric imaging processed can also be used independently, i.e. without creating an anatomical model, to predict the location of an electrode within the anatomical cavity, e.g. for the purposes of tracking an interventional device.

There is an ongoing desire to improve the quality of a mapping function usable for dielectric imaging processes, for instance, to facilitate high quality tracking of the position of an interventional device and/or the generation of more accurate models of the internal anatomy.

### SUMMARY OF THE INVENTION

The invention is defined by the claims.

The present disclosure proposes a processing system for providing a quality measure of a mapping function, wherein the mapping function predicts a position of an electrode, within an anatomical cavity, using the electrical response of the electrode to crossing electric fields induced within the anatomical cavity.

The processing system is configured to: generate at least one quality measure, each quality measure representing a quality of the electrode position predicting performed by the mapping function with respect to a particular portion of the anatomical cavity; and control a user interface to provide a user-perceptible representation of the at least one quality measure.

In other words, the disclosure proposes an approach for providing feedback on the predicted quality of a mapping function, and in particular, to the predicted quality of the mapping function with respect to a particular portion (e.g. part or all) of the anatomical cavity.

It is recognized that the mapping function may have different levels of quality for different parts of the anatomical cavity. For instance, the mapping function may provide highly accurate predictions of the electrode position (e.g. be high-quality) if the electrode is in a first part of the anatomical cavity, and may provide low accuracy predictions of the electrode position if the electrode is in a second part of the anatomical cavity.

Thus, there is a principle of a "local" quality measure, being a quality measure that represents the quality of the mapping function for only a part of the (known) anatomical cavity, and a "global" quality measure, that represent the quality of the mapping function for the entire known anatomical cavity.

The feedback of the at least one quality measure indicates whether a sufficient amount and/or variety of initial data had been collected to generate a high-quality mapping function. In particular, if an insufficient amount and/or variety of initial data has been collected, then the mapping function may have a low quality with respect to a particular portion or portions of the anatomical cavity.

The process for generating a mapping function requires an investigative procedure, in which an interventional device mounting two or more electrodes (e.g. having a predetermined spatial relationship) is moved within the anatomical cavity. Thus, the process of generating a high-quality mapping function requires an invasive procedure, requiring clinician/physician input in order to perform appropriate mapping. By providing a quality measure of the mapping function, a clinician can be guided as to whether sufficient/enough investigation of the anatomical cavity (with the interventional device) has been performed. By providing feedback on the quality of the mapping function, the amount of invasive investigation that a clinician needs to perform can be reduced, whilst reducing a likelihood that insufficient data is collected for generating a high-quality mapping function.

The present disclosure recognizes that provision of a quality measure during the generation of the mapping function is particularly advantageous to reducing an impact on a subject. In some embodiments, the at least one quality measure may be obtained and output as a user-perceptible representation (at a user interface) during an investigative procedure, in which the mapping function is generated based on the electrical responses of two or more electrodes mounted upon an interventional device investigating the anatomical cavity. In this way, the present invention aids a clinician in performing the generation of a high quality mapping function.

The invention relies upon a recognition that current solutions fail to provide a quality measure that can be used to determine whether the movement of the interventional device (during the investigative procedure) is sufficient to obtain appropriate electrical responses for creating a high quality mapping function, e.g. whether the mapping function can predict the position of an electrode accurately, confidently and/or robustly.

The mapping function may be used to generate an anatomical model of the anatomical cavity. In particular, the predicted positions of the electrode(s) may be recorded/collated and used to define the bounds of an anatomical model. Predicting the quality of the (predicting performed by the) mapping function with respect to particular portions of the anatomical cavity is, in these circumstances, functionally equivalent to predicting a quality of the portion of the anatomical model representing that particular portion of the anatomical cavity. This is because the quality of the anatomical model (at each portion) is dependent upon the quality of the mapping function (with respect to that portion).

An anatomical model may be a 3D anatomical model or dataset that facilitates generation of a 3D representation of the anatomical cavity, e.g. allows a 3D representation of the anatomical cavity to be rendered and displayed.

The user interface may be controlled via an output interface. That is, the processing system may comprise an output interface configured to provide output signals for input to the user interface (to thereby control the user interface).

The processing system may be configured to generate at least one quality measure by: obtaining, from a processor that generates the mapping function, the predicted position of two or more electrodes, mounted on an interventional device, within the anatomical cavity for different positions and/or orientations of the interventional device within the anatomical cavity; determining one or more characteristics of the predicted positions of the two or more electrodes based on the determined predicted positions; and determining, based on the one or more characteristics, the quality measure.

This embodiment makes use of characteristics (e.g. statistical characteristics) of predicted positions or "sample points" of electrodes within the anatomical cavity to predict a quality of the mapping function with respect to a particular portion of the anatomical cavity, which will be derived from the predicted positions.

The processing system predicts/determines a predicted position (within an anatomical cavity) of two or more electrodes at a plurality of positions and/or orientations for the interventional device that mounts/supports the two or more electrodes. The predicted position defines a predicted position of an electrode in a multidimensional (e.g. 2D or 3D) co-ordinate space representing the anatomical cavity.

This is achievable because the crossing electric fields induce position dependent responses in electrodes at different positions. This means that each position of the electrode can be associated with a (semi-) unique response of an electrode to the electric field(s). The predicted position can be determined using the known/predetermined positional relationship between the electrodes mounted on an interventional device, to effectively measure or map how the electrical response(s) correspond to a true spatial position.

This embodiment makes use of characteristics of predicted positions to calculate a quality of the mapping function. This recognizes that the mapping quality depends upon the data that has been used to produce and determine the predicted positions.

The processor that generates the mapping function process may be the processing system itself or another processor. This processor may be configured to generate the mapping function by obtaining the responses of two or more electrodes of an interventional device for different positions and/or orientations of the interventional device, using a (template) mapping function to predict the positions of the two or more electrodes for the different positions and/or orientations of the interventional device, and using a known spatial relationship between the two or more electrodes (e.g. known distances between the two or more electrodes) to iteratively adjust/modify/update the mapping function so that predicted positions(s) adhere (within a margin of error and/or acceptability) to the known spatial relationship.

In particular, the obtained predicted positions of the two or more electrodes may comprise (a subset of) the obtained predicted positions for the responses of the two or more electrodes used to generate/update a most recently generated/updated mapping function.

Optionally, the one or more characteristics comprise one or more statistical measures of the predicted positions of the two or more electrodes.

A statistical measure is any measure that summarizes information about the predicted positions. Examples of suitable statistical measures include an average (e.g. mean) distance between the predicted positions, a standard deviation of distances between the predicted positions and so on.

The one or more characteristics may comprise a density of the determined predicted positions. It is recognized that the more sample points that are taken, i.e. the more examples of predicted positions that are determined, the more precise (and therefore higher quality) that an anatomical model reconstructed from the predicted positions will become.

The density may be a local density (e.g. a density with respect to a particular part of the anatomical cavity) and/or a global density, e.g. a density with respect to all predicted positions of the electrode(s).

Optionally, the one or more characteristics comprises a uniformity of the distribution of the predicted positions with respect to the anatomical cavity.

The processing system may be configured to generate at least one quality measure by: obtaining, from a processor generating the mapping function, the predicted position of two or more electrodes, mounted on an interventional device, within the anatomical cavity for different positions and/or orientations of the interventional device within the anatomical cavity; determining one or more characteristics of the different positions and/or orientations of the interventional device based on the determined predicted positions of the two or more electrodes; and determining, based on the one or more characteristics, the quality measure.

The present invention also recognizes that a sufficient quantity and/or variety of interventional device positions and/or orientations are needed to generate a high-quality mapping function.

In particular, predicted positions and/or orientations of the interventional device may be obtained from the predicted positions for the responses of the two or more electrodes (of the interventional device) used to generate a most recently generated/updated mapping function.

In some examples, the step of determining one or more characteristics of the different positions and/or orientations of the interventional device comprises determining, for each position and/or orientation of the interventional device at which electric responses are obtained, an orientation of the interventional device with respect to the anatomical cavity based on the predicted positions for the two or more electrodes at the position and/or orientation of the interventional device; and the one or more characteristics comprises a uniformity of the distribution of the determined orientations of the interventional device.

It is herein recognized that the distribution, i.e. range or variety, of angles (of the interventional device) over which the electrode responses are made has a significant impact on the quality of any reconstructed anatomical model. This is because when a specific angle or region in the cavity is disproportionally present in the data, the generation of the mapping function will be biased towards that orientation/region - i.e. produces an overfitted mapping function.

The processing system may be configured to generate at least one quality measure by determining a sensitivity of the mapping function to changes in the obtained electrical responses.

The processing system may be configured to determine a sensitivity of the mapping function by: processing sample electrical responses using the mapping function to determine first sample predicted positions of the electrodes; modifying the sample electrical responses; processing the modified sample electrical responses using the mapping function to determine second sample predicted positions of the electrodes; and determining a sensitivity of the mapping function based on the first sample predicted positions and the second sample predicted positions.

Above examples generally generate the at least one quality measure based on the predicted position(s) of electrodes, the predicted positions being generated using the mapping function. In some examples, at least one quality measure is derived from input data used to generate the mapping function, e.g. from the electrical responses.

In one example, the processing system is configured to determine one or more statistical characteristics or measures of the electrical responses. Examples of suitable statistical measures include an average (e.g. mean) difference between the electrical responses, a standard deviation of differences between the electrical responses and so on. The statistical characteristics can be used to generate a quality measure, in a similar manner to the statistical characteristics of the predicted positions as previously described.

In some examples, the processing system is configured to generate at least one quality measure by determining an accuracy of obtained electrical responses of the two or more electrodes. The accuracy of the mapping function will be dependent upon the accuracy of the electrical responses used to generate the mapping function. Thus, if the obtained electrical responses are inaccurate, e.g. due to noise or an erroneous/broken electrode, then it can be inferred that the mapping function itself is also inaccurate.

The step of determining a quality measure may comprise determining a global quality measure representing the predicted overall quality of the mapping function for predicting the position of an electrode within the entire known anatomical cavity.

The step of determining a quality measure may comprise determining one or more local quality measures, each local quality measure representing a predicted quality of the mapping function for predicting a position of an electrode only a part of the (known) anatomical cavity.

In some examples, the processing system is further configured to generate guidance information for guiding a user to reposition and/or reorient the interventional device at a location of the anatomical cavity based on the determined one or more local quality measures.

The processing system may be adapted wherein the processing system is configured to identify, based on the determined one or more local quality measures, a part of the anatomical cavity associated with the lowest quality position prediction performed by the mapping function; and the guidance information is designed for guiding a user to reposition the interventional device based on the identified part of the anatomical cavity.

In some examples, the processing system can be adapted wherein the guidance information comprises guidance to reposition the interventional device such that there is maximum information gain for the mapping function, that is, a position which is predicted to yield the maximum increase of quality. The position may, for instance, be identified using a machine-learning method or the like or be identified to meet some desired statistical propertes for the predicted positions.

The processing system may further comprise the user interface for displaying a visual representation of the quality measure(s).

There is also proposed a processing arrangement for providing a mapping function for predicting the position of an electrode within an anatomical cavity. The processor arrangement may comprise a processing system as previously described and a mapping function generator.

The mapping function generator is configured to obtain, e.g. at an input interface, a response from two or more electrodes to the crossing electric fields. The mapping function generator is configured to process the obtained responses to generate the mapping function, e.g. by iteratively modifying a mapping function to adhere to a known spatial relationship between the two or more electrodes within a predetermined margin of error.

The processing system as previously described may be configured to generate one or more quality measures for the mapping function generated by the mapping function generator.

The processing arrangement may further comprise a dielectric imaging processor configured to generate an anatomical model of an anatomical cavity by processing the predicted positions of two or more electrodes within the anatomical cavity.

The processing arrangement may further comprise an interventional device that mounts the two or more electrodes.

There is also proposed a computer-implemented method for providing a quality measure of a mapping function, wherein the mapping function predicts a position of an electrode, within an anatomical cavity, using the electrical response of the electrode to crossing electric fields induced within the anatomical cavity.

The computer-implemented method comprises: generating at least one quality measure, each quality measure representing a quality of the electrode position predicting performed by the mapping function with respect to a particular portion of the anatomical cavity; and controlling a user interface to provide a user-perceptible representation of the at least one quality measure.

The computer-implemented method may be configured to generate at least one quality measure by: obtaining, from a processor that generates the mapping function, the predicted position of two or more electrodes, mounted on an interventional device, within the anatomical cavity for different positions and/or orientations of the interventional device within the anatomical cavity; determining one or more characteristics of the predicted positions of the two or more electrodes based on the determined predicted positions; and determining, based on the one or more characteristics, the quality measure.

The computer-implemented method may be configured to generate at least one quality measure by: obtaining, from a processor generating the mapping function, the predicted position of two or more electrodes, mounted on an interventional device, within the anatomical cavity for different positions and/or orientations of the interventional device within the anatomical cavity; determining one or more characteristics of the different positions and/or orientations of the interventional device based on the determined predicted positions of the two or more electrodes; and determining, based on the one or more characteristics, the quality measure.

The computer-implemented method may be adapted to generate at least one quality measure by determining a sensitivity of the mapping function to changes in the obtained electrical responses. Determining a sensitivity may be performed by: processing sample electrical responses using the mapping function to determine first sample predicted positions of the electrodes; modifying the sample electrical responses; processing the modified sample electrical responses using the mapping function to determine second sample predicted positions of the electrodes; and determining a sensitivity of the mapping function based on the first sample predicted positions and the second sample predicted positions.

The computer-implemented method may be configured so that the step of determining a quality measure comprises determining a global quality measure representing the predicted overall quality of the mapping function for predicting the position of an electrode within the entire known anatomical cavity.

The computer-implemented method may be configured so that the step of determining a quality measure comprises determining one or more local quality measures, each local quality measure representing a predicted quality of the mapping function for predicting a position of an electrode within only a part of the anatomical cavity.

There is also proposed a computer program product comprising computer program code means which, when executed on a computing device having a processing system, cause the processing system to perform all of the steps of any herein claimed method.

Similarly, there is also proposed a computer-readable (storage) medium comprising instructions which, when executed by a computer or processing system, cause the computer or processing system to carry out (the steps of) any herein described method. There is also proposed computer-readable data carrier having stored thereon the computer program (product) previously described. There is also proposed a data carrier signal carrying the computer program (product) previously described.

The skilled person would be readily capable of adapting any herein described method to reflect embodiments of herein described apparatus, systems and/or processors, and vice versa. A similar understanding would be made by the skilled person with respect to a computer program (product).

Additional aspects, features, and advantages of the present disclosure will become apparent from the following detailed description.

### BRIEF DESCRIPTION OF THE DRAWINGS

For a better understanding of the invention, and to show more clearly how it may be carried into effect, reference will now be made, by way of example only, to the accompanying drawings, in which:
Figure 1 illustrates positions for a set of external electrodes positioned on a subj ect;
Figure 2 illustrates a co-ordinate system for defining a direction of electric fields generated by the external electrodes;
Figure 3 illustrates a dielectric imaging system;
Figure 4 illustrates an approach for reconstructing an anatomical model from a point cloud;
Figure 5 illustrates a method according to an embodiment;
Figure 6 illustrates a method for use in an embodiment;
Figure 7 illustrates a display of quality measures;
Figure 8 illustrates a method according to an embodiment;
Figure 9 illustrates a display of guidance information; and
Figure 10 illustrates a processing system according to an embodiment.

### DETAILED DESCRIPTION OF THE EMBODIMENTS

The invention will be described with reference to the figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the figures are merely schematic and are not drawn to scale. It should also be understood that the same reference numerals are used throughout the figures to indicate the same or similar parts.

The invention provides a mechanism for generating and providing one or more quality measures of a mapping function (for mapping electrical responses of an electrode to a predicted position of that electrode within an anatomical cavity) to a user, such as a clinician. Each quality measure predicts or indicates a quality of a mapping function with respect to a particular part of an anatomical cavity, for instance, indicating a predicted accuracy of the mapping function for predicting a position of an electrode located within a particular portion of the anatomical cavity. A user-perceptible output is provided that indicates the quality measure for the user.

The present disclosure is based on the realization that generation of an accurate and reliable mapping function can be ensured by providing one or more quality measures of the mapping function. In particular, as generation of a mapping function requires an invasive procedure, there is a strong incentive to minimize potential damage to the subject/patient being imaged, whilst still ensuring that a highly accurate mapping function is generated. The present disclosure recognizes that the provision of a quality measure to a user provides them with useful feedback for constructing a mapping function that minimizes potential damage to the subject.

Embodiments of the invention may be employed in any suitable scenario in which a mapping function for mapping a response of an electrode (to crossing electric fields) to a predicted position with an anatomical cavity is required. One example scenario is in the generation of an anatomical model of the anatomical cavity or for the tracking of the position of an interventional device within the anatomical cavity.

For the purposes of contextual understanding, the principle and purpose of inducing electric fields within a subject will be hereafter described.

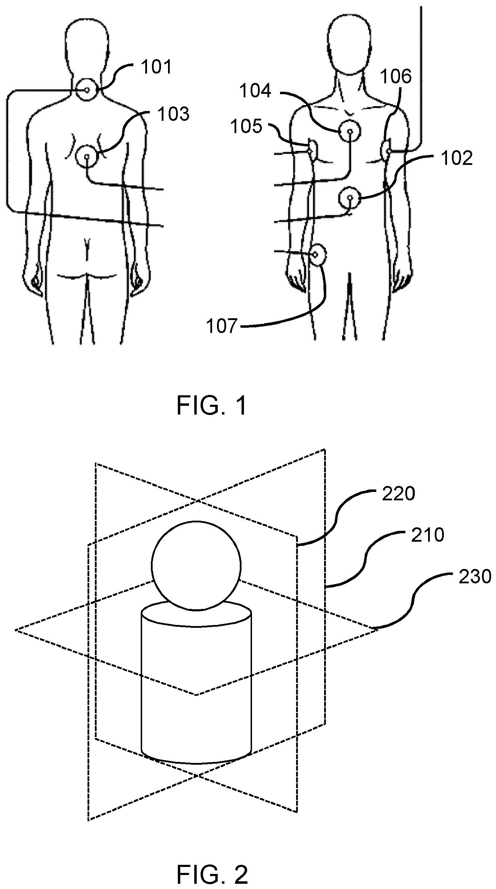

Figure 1 illustrates positions for a set of external electrodes positioned on a subject 190. External electrodes are electrodes that are positioned externally to a subject, and are contrasted with internal electrodes that are positionable within the subject.

The illustrated set of external electrodes comprises a first external electrode 101, a second external electrode 102, a third external electrode 103, a fourth external electrode 104, a fifth external electrode 105 and a sixth external electrode 106. The illustrated set further comprises a reference electrode 107, which can act as a "ground" for defining a base/background level of electric field activity in the subject.

An electric signal provided to each external electrode is controlled to thereby define crossing electric fields between the electrodes. In particular, the first 101 and second 102 external electrodes form a first pair of external electrodes ("external electrode pair"), and signals provided to the first external electrode pair can be controlled to induce a first electric field therebetween. The third 103 and fourth 104 external electrodes form a second pair of external electrodes, and signals provided to the second external electrode pair can be controlled to induce a second electric field therebetween. The fifth 105 and sixth 106 external electrodes form a third pair of external electrodes, and signals provided to the third external electrode pair can be controlled to induce a third electric field therebetween.

Electric signals provided to each external electrode, and in particular to each pair of electrodes, can control the frequency and/or magnitude/strength of the crossing electric fields. The electric signals supplied to the electrodes may be controlled by an electric field generator (not illustrated in Figure 1).

The crossing electric fields are usable to track or identify a location of electrodes positioned within the crossing electric fields. In particular, a response of an electrode to each electric field will change as a position within the electric field changes (e.g. a distance from a source/sink of the electric field changes). The response of an electrode to the crossing electric fields can be mapped or associated with a particular position within the electric fields.

In other words, an electrical response (which can be alternatively labelled a "measurement") of the internal electrode to the crossing electric fields can be processed to determine a predicted position of the internal electrode (e.g. and therefore of any interventional device comprising the internal electrode) within an anatomical cavity of the subj ect.

In particular, each electric field may be controlled to have a particular/different frequency. This facilitates determination of the predicted position of an electrode with respect to each electric field, by assessing the response of the electrode to the particular frequency of the electric field. This information can be used to effectively identify or predict the position of the electrode with respect to a co-ordinate system defined by the electric fields.

One of the external electrode pairs may be omitted, e.g. if only two crossing electric fields are desired (e.g. for performing a 2-dimensional tracking process).

Figure 2 schematically illustrates a co-ordinate system for defining a direction of electric fields generated by the external electrodes. The co-ordinate system defines three cardinal planes 210, 220, 230. The crossing electric fields may, for instance, be optimally positioned when the direction of each electric field is parallel to a respective cardinal place 210, 220, 230.

The intent of the crossing electric fields, e.g. such as those generated using the set illustrated by Figure 1, is to facilitate identification of the position of an electrode (or group of electrodes) with respect to a co-ordinate system, such as that illustrated by Figure 2.

A more complete example of how to track the position of an internal electrode with respect to crossing electric fields (i.e. within the subject), and optionally how to further exploit this information, e.g. for the construction of an anatomical model of an anatomical cavity of the subject, is hereafter described for the purposes of improved contextual understanding.

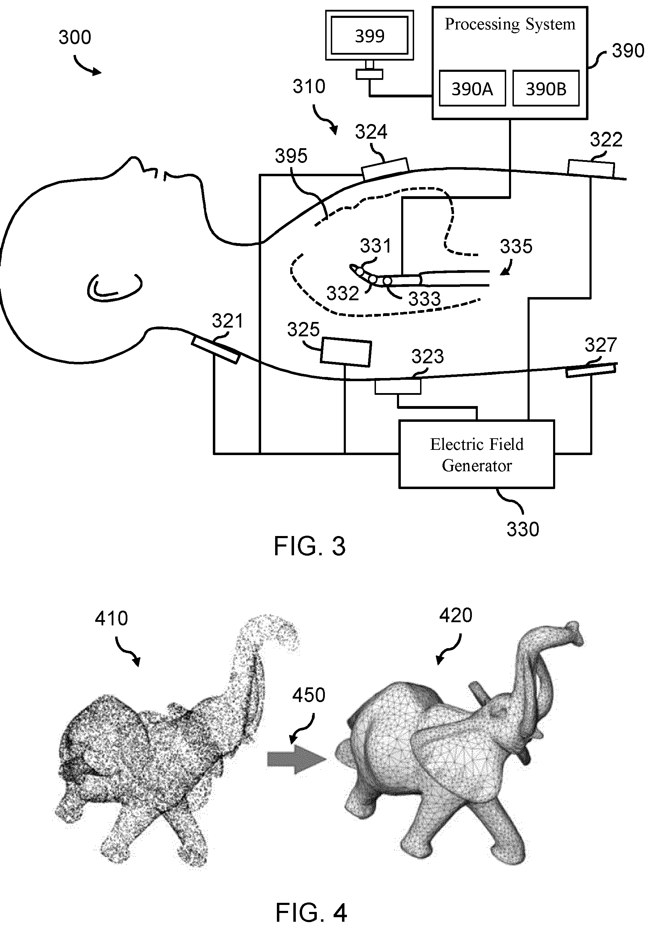

Figure 3 conceptually illustrates a processing arrangement 300 for generating a mapping function for predicting the position of an electrode within an anatomical cavity. The processing arrangement 300 may further generate an anatomical model of an anatomical cavity within a subject.

In particular, the processing arrangement 300 may be configured to generate an anatomical model (of an anatomical cavity, such as a blood vessel and/or chamber) using a dielectric imaging process.

The dielectric imaging system 300 comprises an exemplary electric field generating arrangement 310 and a processing system 390. The processing system 390 may contain a mapping function generator 390A (i.e. a "processor") and/or a dielectric imaging processor 390B, although these may be alternatively positioned externally to the processing system 390.

The electric field generating arrangement 310 comprises a set of external electrodes 321, 322, 323, 324, 325, 327 for positioning positioned externally with respect to the subject 390 (e.g. as electrode patches provided on a skin of the subject). The set 310 of external electrodes may comprise a plurality of electrode pairs angled with respect to one another (e.g. positioned orthogonally to one another), so that any electric fields generated by the electrode pairs are angled with respect to one another. These electrode pairs may comprise a first electrode pair (formed of a first 321 and second 322 external electrode), a second electrode pair (formed of a third 323 and fourth 324 external electrode) and a third electrode pair (formed of a fifth 325 and sixth (not visible) external electrode). One of more of these electrode pairs may be omitted. The set of external electrodes may also comprise a reference electrode 327. Thus, the external electrodes are placable in a similar manner to the set of external electrodes illustrated in Figure 1.

The electric field generating arrangement 310 also comprises an electric field generator 330, which is adapted to control (characteristics of) an electric signal (e.g. voltage and/or current) supplied to each external electrode.

The electric field generating arrangement 310 is configured to generate a plurality of (here: three) crossing (intra-body) electrical fields using the external electrodes. This is performed using appropriate control of the electric signals provided to each external electrode.

In particular, each electrode pair may be appropriately controlled to induce an electric field between each electric pair. Thus, where there are three electrode pairs, three electric fields may be generated. Preferably, the frequency of each generated electric field is controlled to be different, to facilitate increased ease in identifying a relative location of an electrode positioning within the crossing electric fields.

The generated electric fields can be used to define or establish a relative location of an (internal) electrode positioned within the crossing electric fields. In particular, as previously explained, the (electrical) response of an internal electrode to the electric fields changes as the predicted position of the internal electrode moves about the subject. This is at least partly because the induced electrical fields' distribution is inherently inhomogeneous due to the different dielectric properties and absorption rates (related to conductivity) of the interrogated tissues. The principle of crossing electric fields thereby facilitates tracking of the predicted position of the internal electrode using a dielectric imaging processor 390 that monitors a response of any internal electrodes to the crossing electric fields, e.g. by mapping the (electrical) response of an electrode to a predicted position within the subject or using a suitable mapping/transfer function, e.g. the "V2R function" described below to determine the predicted position(s) of the electrodes.

By way of further explanation, for the purposes of improved conceptual understanding, if the crossing electric fields are controlled to have a different frequency with respect to one another, then the response of the internal electrode to each frequency could be used to determine a relative distance between from each source/sink of the corresponding electric field. This principle can be used to effectively triangulate the predicted position of the internal electrode within the crossing electric fields.

For instance, if there are three external electrode pairs, positioned to emit electric fields (E₁, E₂, E₃) of different frequencies that are angled (e.g. near-orthogonal) with respect to one another, a voltage response (V₁, V₂, V₃) of an internal electrode (identifying a voltage (e.g. between the electrode and the reference electrode or between the electrode and the electrode generating the electric field) at each of these three frequencies) will differ depending upon position within the anatomical cavity.

The skilled person will appreciate that determining the position of internal electrodes 331, 332, 333 also facilitates determining the position, orientation and/or angle of an interventional device 325 that mounts the electrodes. In particular, a positional relationship of the electrodes on the interventional device may be known/predetermined, and used to derive an orientation of the interventional device (e.g. define an axis along which the interventional device lies).

Other forms of response for an internal electrode (e.g. an impedance response or a capacitive response, e.g. indicating change in impedance/capacitance between the internal electrode and an external electrode) will be apparent to the skilled person.

The electric field generator 330 may be configured to control the electric fields, generated using the set of external electrodes, to operate in the frequency range of 30-100 kHz. This frequency range is particularly useful for ensuring penetration of a subject, whilst providing a frequency range that attenuates in human tissue without significant damage/injury to the tissue. The reference electrode serves as an electric reference for all measurements taken by electrodes, e.g. as a reference for the response of the internal electrodes.

The response of two or more internal electrodes mounted upon a single interventional device can be used to construct (and update) the mapping function used to map a response of the internal electrode to a predicted position within the anatomical cavity (e.g. within some Euclidian/Cartesian space).

An exemplary process for generating or constructing a mapping function using a mapping function generator 390A is hereafter described.

As previously mentioned, the mapping function generator makes use of a plurality of internal electrodes 331, 332, 333 to be positioned within the anatomical cavity (e.g. electrodes positioned on an interventional device 335 to be inserted into the anatomical cavity). A spatial relationship (e.g. a distance) between each of the internal electrodes may be predetermined and/or known.

The mapping function generator 390A is configured to receive (and optionally provide) signals (e.g. at an input interface) from/to the internal electrodes 331, 332, 333 to determine a response of the internal electrodes to the crossing electric fields.

The response of the internal electrodes (e.g. to externally applied electric fields by the external electrodes) is then iteratively recorded for different positions and/or orientations of the interventional device, i.e. to obtain a "measurement" from each internal electrode. The mapping function generator can then repeatedly define/update and apply a mapping function or transfer function ("V2R function") that transforms each recorded response to Euclidian/Cartesian coordinates (R-space), whilst ensuring known properties and/or spatial relationships of the internal electrodes and/or interventional device 335 (e.g. electrode spacing and electrical weight length) as well as a set of other constraints are maintained. For instance, the mapping/transfer function can effectively learn and linearize the distorted relation between the measured responses and the actual position in three dimensions by taking the known inter-electrode distances as a reference. By way of example only, if an internal electrode has a response matching a previously measured response then the relative distance to the responses measured by the other internal electrodes can be determined and used to improve the mapping/transfer function.

In other words, the mapping/transfer function effectively determines or predicts a relative/predicted location of each electrode in a Euclidian/Cartesian and/or multidimensional space or co-ordinate system ("R-space"). In this way, an R-space cloud of points (known Euclidian/Cartesian co-ordinates) can be built up and updated as the internal electrodes are moved within the anatomical cavity. This R-space cloud of points is then iteratively analyzed in order to iteratively update the mapping function to adhere to the known properties of the internal electrodes and/or interventional device, such as a spatial relationship of the internal electrodes.

Other descriptions and/or embodiments of the process for generating the mapping function are disclosed by the European Patent Applications EP 0775466 A2, EP 3568068 A1 and EP 3607879 A1.

The predicted positions of an internal electrode or internal electrodes may also be used to construct an anatomical model of an anatomical cavity (i.e. a cavity in which the interventional device is able to move). This process is called a dielectric imaging process, and may be performed by the dielectric imaging processor 390B.

Broadly, the dielectric imaging processor 390B may build an anatomical model of an anatomical cavity 395 (e.g. a chamber, vessel or void) within a subject by processing an R-space cloud of points.

In particular, using an updated R-space cloud of points, such as the R-space cloud of points produced during the construction of the mapping function, a reconstruction algorithm generates an anatomical model of the (investigated part of the) anatomical cavity. The anatomical model may, for example, be a 3D surface that depicts or models the (known bounds of) the anatomical cavity.

The process of reconstructing an anatomical model from a point cloud is conceptually illustrated in Figure 4, which demonstrates a process 450 in which a cloud of R-space points 410 ("point cloud") is transformed into an anatomical model 420. In the illustrated example, this is performed by creating a (3D) surface from the point cloud data, methods of which will be readily apparent to the skilled person.

For example, a point cloud can be converted into a polygon mesh or triangular mesh model (or other surface model) using a surface reconstruction approach. A variety of suitable approaches is discussed in Berger, Matthew, et al. "A survey of surface reconstruction from point clouds." Computer Graphics Forum. Vol. 26. No. 1. 2017, and further mechanisms will be readily apparent to the skilled person.

In this way, the "global field" measurements (i.e. the effect of the electric fields generated by external electrodes on the internal electrodes) can be used to generate a rough anatomical model of the anatomical cavity.

Referring back to Figure 3, more precise identification of the bounds and features of the anatomical model can be performed by monitoring the response of the internal electrodes 331, 332, 333 to local electric fields (e.g. fields generated by other internal electrodes). Thus, in some embodiments, the dielectric imaging processor 390 may also control the internal electrodes 331, 332, 333 to generate an electric field (which can be detected by other internal electrodes). The response of the internal electrodes to local electric fields can be labelled "local field measurements", as compared to the response of the internal electrodes to externally induced electric fields which can be labelled "global field measurements".

For instance, changes in a local electrical field (being an electric field induced between two internal electrodes 331, 332, 333) can indicate the presence or absence of tissue between the two internal electrodes. A response of an internal electrode to a local electric field can therefore be used to identify the presence or absence of tissue, to thereby facilitate tuning or updating of the anatomical model.

In some examples, additional processing of global field measurements (i.e. the responses of the internal electrodes to electric fields induced by the external electrodes) can be performed to improve the precision of the bounds and features of the anatomical model.

For example, regions with inherently marked steep gradients in the electrical field responses can be identified. It is recognized that such regions indicate the bounds of the anatomical cavity and/or other information, e.g. drainage of vessels into or out of a cardiac chamber as well as the valves of a cardiac chamber. These features can thereby be uniquely identified by the system and imaged even without physically visiting them with the interventional device 335.

The combined global and local field measurements enable sophisticated detection and effective handling of inconsistencies and outliers, level of electrode shielding/coverage (e.g. by measuring location inter-correlation), pacing (saturation), as well as physiological drift. Drift can, for example, be detected using a moving window over time and corrected continuously whereby the internal electrode location remains accurate throughout the whole procedure making the system resilient to drift.

The above-provided description of a dielectric imaging process is only an example, and the skilled person would be readily capable of modifying the described process appropriately.

The skilled person will appreciate that the derived mapping/transfer function ("V2R function") can also be used to track a location of the internal electrodes with respect to a constructed anatomical model. This facilitates the generation and display of an anatomical model and an indicator of a current location/position of an interventional device 335 mounting internal electrodes 331, 332, 333 with respect to the anatomical model.

Of course, a visual representation of any generated anatomical model and/or determined position may be generated and provided at a user interface 399.

The present disclosure relies upon a concept of generating one or more quality measures that predict a quality of the mapping function, which are then displayed to a user (e.g. a clinician controlling the dielectric imaging process). Predicting a quality of the mapping function also predicts a quality of the anatomical model generated using the mapping function, as the quality of the anatomical model is dependent upon at least a quality of the mapping function.

The process of generating the mapping function is an invasive procedure, as it effectively requires repeatedly probing an anatomical cavity to obtain a sufficient number and/or variety of measurements to generate a mapping function for the anatomical cavity. There is a strong desire to minimize the time spent gathering data needed to perform the dielectric imaging process and/or a device tracking process.

In this context, the inventors have recognized that an indication as to a quality of the mapping function provides useful clinical information to help guide the clinician to perform sufficient investigation of an anatomical cavity to facilitate generation of a high-quality mapping function.

Various approaches for generating a quality measure are envisaged by the present invention, such as those hereafter described.

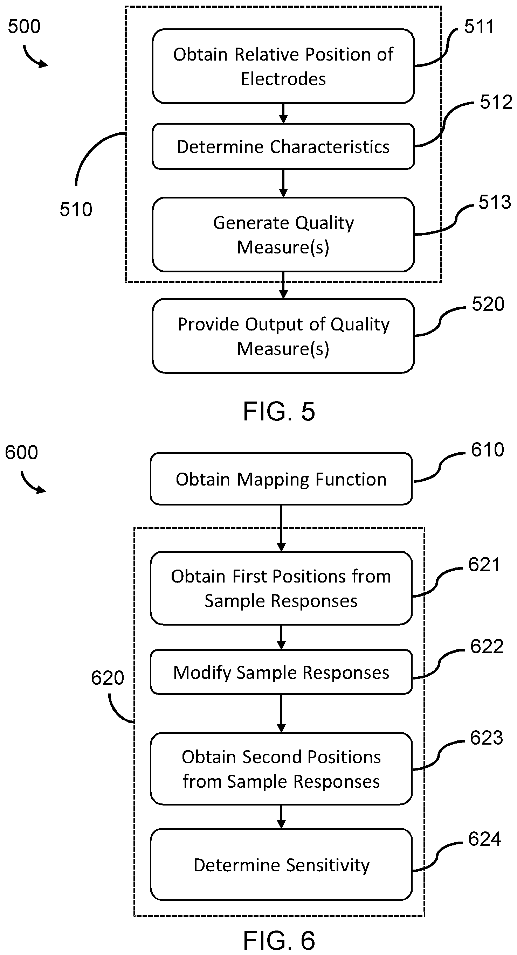

Figure 5 illustrates a (computer-implemented) method 500 according to an embodiment. The method 500 is carried out by a processing system according to an embodiment, e.g. the processing system 390 of Figure 1.

The method 500 comprises a process 510 of generating at least one quality measure, each representing/predicting a quality of the electrode position predicting performed by the mapping function with respect to a particular portion of the anatomical cavity.

The quality measure(s) may comprise a global quality measure, e.g. representing a quality of the overall mapping function (i.e. a quality of the mapping function with respect to the entire known anatomical cavity). The quality measure(s) may additionally or otherwise comprise one or more position-dependent ("local") quality measures, e.g. representing a quality of the mapping function with respect to its ability to predict a position of the electrode in a particular part of the anatomical cavity. A local quality measure is therefore associated with a particular part or sub-region of the anatomical cavity.

A global quality measure may be generated using information derived from electrical responses of the electrodes positioned at any (predicated) position within the investigated part of the anatomical cavity. A local quality measure may be generated using information derived from electrical responses of the electrodes positioned at any (predicted) position within a certain area/part of the investigated part of the anatomical cavity.

Thus, a local quality measure is associated with a particular part of the anatomical cavity. A global quality measure may be associated with the known entirety of the anatomical cavity (i.e. an investigated entirety of the anatomical cavity).

The method 500 also comprises a step 520 of controlling a user interface to provide a user-perceptible representation of the at least one quality measure. The processing system may itself comprise the user interface, or may communicate with the user interface using output signals (e.g. provided at an output interface of the processing system).

Any suitable mechanism for communicating a user-perceptible representation of the at least one quality measure can be used, e.g. using an audio, visual or haptic output. Examples include the use, for a visual output, of a scale-bar, a color, an indicator, textual information and so on and for an audio output, a sound or beep. More detailed and specific examples will be provided later.

It is appreciated that the quality measure(s) may change over time. For instance, a quality of the mapping function may increase with the number of measurements collected from the interventional device. A decrease in quality may result from changes in the adjustments to the direction of one or more of the electric fields, e.g. due to changes in the position of the electrodes, or when the anatomical structure changes during longer procedures, e.g. as a result of ablation, tissue removal or when entering additional un-mapped areas.

Thus, in some examples, steps 510 and 520 are iteratively repeated, to consistently and repeatedly assess and update the visual representation of the at least one quality measure.

Various approaches for performing step 510 are envisaged by the present invention, of which one is illustrated by Figure 5.

Generally, quality can be divided into at least three aspects or "types": accuracy, confidence and robustness/sensitivity. A quality measure may provide a value/indicator representing one or more of these types/properties, and different quality measures may provide measures of different types of quality.

An accuracy may indicate a predicted/absolute error of predicted positions of electrodes, which would impact on the accuracy of any mapping function generated by processing predicted positions of the electrodes.

A confidence may indicate a risk of error in the mapping function, e.g. a risk that an insufficient amount and/or variety of data has been collected to construct an accurate mapping function. An insufficiently confident mapping function may be prone to error, e.g. is more likely to inaccurate. A confidence may be calculated by calculating statistical information of predicted positions of the electrodes and/or the positions/orientations of an interventional device mounting the electrodes to ensure that a sufficient amount/variety of data is collected.

A sensitivity may indicate a sensitivity in a mapping function to changes in input data. A highly sensitive mapping function indicates a probable high-error of the mapping function to correctly predicting the position of an electrode.

A number of mechanisms for determining a quality measure for a mapping function based on these principles is hereafter described, although the proposed concepts are not considered exhaustive.

In the illustrated example, step 510 comprises a step 511 of obtaining, from a processor that generates the mapping function, the predicted position of two or more electrodes, mounted on an interventional device, within the anatomical cavity for different positions and/or orientations of the interventional device within the anatomical cavity. The predicted positions may be obtained at a user interface of the processing system.

A predicted position is a position of the internal electrode with respect to the crossing electric fields as calculated using the mapping function. Thus, the predicted position may be a determined position of an electrode within the crossing electric fields as defined by a Euclidian/Cartesian co-ordinate space.

In particular, the obtained predicted positions of the two or more electrodes may comprise only predicted positions generated based on the responses of the two or more electrodes used to generate/update a most recently generated/updated mapping function.

For generating a local quality measure, step 511 only obtains the predicted positions of two or more electrodes when the positions fall within a particular area or region of the investigated part of the anatomical structure. Thus, in some examples, only a subset of all possible positions for the electrodes are obtained.

For a global quality measure, step 511 may comprise obtaining all recorded positions or a representative sample of all recorded positions.

In some examples, for a global quality measure, step 511 may comprise obtaining only a subset of all recorded positions. For instance, an algorithm may be used to automatically select a range of recorded positions (e.g. for a selected range of positions/orientations of the interventional device), e.g. a subsample that provides a more even distribution of the electrodes and/or positions and/or orientations of the interventional device. This may facilitate generation of a quality measure that reflects how well subsampling could be used to provide a higher quality mapping function.

The illustrated step 510 also comprises a step 512 of determining one or more characteristics of the predicted positions of the two or more electrodes based on the determined predicted positions. The illustrated step 510 also comprises a step 513 of determining, based on the one or more characteristics, the quality measure. The quality measure may be a value and/or other indicative data (e.g. a binary indicator, a categorical indicator and so on) that is responsive to changes in the quality of the mapping function.

In another example, step 512 comprises determining one or more characteristics of the different positions and/or orientations of the interventional device based on the determined predicted positions of the two or more electrodes. In this scenario, step 513 may still comprise determining, based on the one or more characteristics, the quality measure.

Thus, the quality measure may be based upon characteristics, and preferably statistical characteristics, of the determined positions of internal electrodes and/or the interventional device that mounts the interventional device. It will be appreciated that any suitable position-based characteristics or statistical measures that affect a quality of the mapping function could be used as the characteristics for the sake of steps 512 and 513.

The characteristics may include, for instance, a determined accuracy of the (predicted) positions of the two or more electrodes. This determined accuracy may represent a predicted error of the position of the electrodes at a particular location of the interventional device. Thus, step 512 may comprise determining an accuracy of the positions of the two or more electrodes, e.g. generating one or more accuracy values/measures.

An "accuracy" may comprise a value or number that changes as an accuracy of the positions of the two or more electrodes change, e.g. may be an accuracy value/measures.

In a relatively simple example of calculating an accuracy value/measure, for the purposes of step 512, a position of each of two or more electrodes mounted on an interventional device is predicted, e.g. by processing the response of each electrode to the crossing electric fields using the mapping function. A difference between the positions may then be determined, e.g. to establish a predicted distance between the predicted positions of the electrodes. The predicted distance(s) may be compared to a known or predetermined distance between the electrodes (e.g. based on a known positional relationship between the electrodes on the interventional device), and used to determine an accuracy of the determined positions of the electrodes. One example of such an accuracy is a value responsive to (e.g. equal to, or a sum/average of) the difference between the predicted distance(s) and the known distance(s) for multiple electrodes, with multiple differences being calculated, each being a difference of the predicted distance(s) and known distance(s) for each of a plurality of positions of the interventional device.

Thus, a quality measure may be a measure of accuracy of the positions of electrodes, which can be derived by comparing a predicted distance between electrodes (according to a device tracking system) to a known distance between the electrodes.

It is possible to average the difference between the predicted distance and known distance for multiple electrode groups, to reduce the impact of noise.

Thus, in some examples, step 512 comprises determining one or more distance errors between the predicted positions of two or more electrodes, e.g. based on a known distance between the two or more electrodes. Step 512 may then average the determined distance errors, to determine an accuracy measure.

The determined accuracy (measure) may then be processed, in step 513, to generate the quality measures. For instance, the quality measure may indicate whether or not a determined accuracy measure exceeds some predetermined threshold, and/or provide a cumulative/average determined accuracy. In other examples, each quality measure is equal to a determined accuracy measure. Other suitable mechanisms will be readily apparent to the skilled person.

Step 513 may comprise, for example, deriving a quality measure by processing the accuracy measure and a known size of the electrodes (or known distance between the electrodes). In particular, the quality measure may indicate a relative size of the accuracy measure to the size of the electrodes, e.g. be calculated by dividing the magnitude error by a size of one of the electrodes or the distance between two of the electrodes. Providing a relative measure might provide more useful information to a user than an absolute number. This is because the error might depend on the electrode sizes and/or electrode distances.

As another example, the predicted positions of electrodes mounted on the interventional device can be used to derive statistical information about the predicted positions of the electrodes and/or the interventional device. This statistical information, which forms the one or more characteristics, can be used to determine the quality measure(s). In particular, the statistical information may be used to determine a quality measure representative of a confidence of the mapping function (i.e. that a sufficient amount and/or variety/range of data has been obtained).

One suitable example of statistical information is an evenness/uniformity of distribution of the determined positions of the electrode(s). The present disclosure recognizes that the accuracy of a mapping function is dependent upon how evenly distributed the determined positions (used when generating/updating the mapping function) are throughout the anatomical cavity, i.e. whether a sufficiently large variety/range of positions has been obtained.

Thus, step 512 may comprise determining a (measure of) uniformity of the distribution of the determined positions with respect to the anatomical cavity. The measure of uniformity may, for instance, be a measure of variance and/or skewness in the distribution of the predicted positions.

The measure of uniformity can be processed and/or used to determine one or more quality measures. For instance, a quality measure indicating a uniformity of the measured positions throughout the investigated part of the anatomical structure could be generated, and/or one or more quality measures each indicating a uniformity of positions within a particular region or part of the investigated part of the anatomical structure could be generated.

Another example of statistical information is a density of the determined position(s) of the electrode. The present disclosure recognizes that the more measurements that are taken within a particular area of the anatomical structure, then the more accurate that a mapping function (generated based on these measurements) will be for that area of the anatomical structure. In particular, a relatively low number of measurements would mean that the mapping function might not be stable and prone to change. However, it is also recognized that if there is an extremely large density of measurements for a particular area of the anatomical structure, then the mapping function may again become inaccurate for that area of the anatomical structure, due to overfitting.

Thus, a density of measurements for a particular portion of the anatomical structure is a good indicator of the potential accuracy of the mapping function for said portion of the anatomical structure.

One option for generating a quality measure is to therefore determine a number or density of measurements taken for a particular area (e.g. part or all) of the investigated part of the anatomical structure by processing the determined positions of the electrode(s).

Thus, step 512 may comprise determining a measure of density of the determined positions with respect to a particular region (e.g. part of all) of the investigated anatomical cavity. Multiple density measures may be generated for different areas or parts of the investigated anatomical cavity, and may include a global density measure (indicating a density within the entire investigated part of the anatomical cavity) and/or one or more local density measures (indicating a density within only part of the investigated part of the anatomical cavity).

Step 513 then generates the quality measure(s) based on the density of the determined positions(s) of the electrode(s), e.g. based on the determined measure(s) of density.

In some examples, a quality measure is equal to a density measure. In other examples, a quality measure is equal to a sum/average of density measures.

In another example, a comparison may be made between the density (measure) and a predetermined value to decide whether a sufficient number of measurements have been taken (for that particular part of the investigated part of the anatomical structure or for the whole of the investigated part of the anatomical structure). The determined quality measure may be responsive to this determination.

In another example, step 512 comprises determining a relative orientation/angle of the interventional device at a plurality of positions for the interventional device, based on the determined positions of the electrodes. The determined orientation and/or angle can be determined based on the predicted positions of the electrodes, as previously described.

Statistical information of the relative orientation/angle of the interventional device at a plurality of positions may then be determined, and used to generate a quality measure.

One example of statistical information of the relative orientation/angle of the interventional device is be a measure of uniformity of the distribution of the determined orientations/angles of the interventional device, e.g. a spread of the angles/orientations of the interventional device. This uniformity may be calculated for a local area (e.g. a spread of angles/orientations within a particular region or part of the investigated part of the anatomical structure) and/or for a global area (e.g. a spread of angles/orientation within a particular region or part of the investigated part of the anatomical structure).

One example of a measure of uniformity (of the distribution) is a variance of the determined relative orientations/angles.

In another example, determining a uniformity may comprise calculating a proportion (e.g. a percentage or fraction) of the determined positions and/or orientations that are primarily oriented to lie within the direction of each electric field or in a direction of each axis of multidimensional co-ordinate system (e.g. an axis of the R-space). A difference between the highest and lowest proportion (e.g. a highest and lowest percentage or a highest and lowest fraction) provides a measure of uniformity of the determined angles/orientations.

The present disclosure recognizes that a greater range of angles (for the interventional device) from which measurements are taken increases an accuracy of the mapping function. In particular, a relatively low range of angles could mean that the mapping function might not be stable and prone to change during the mapping process.

Figure 6 illustrates another approach or process 600 to determining a quality measure, e.g. another approach for performing process 510 indicated in Figure 5. The process 600 effectively determines a sensitivity of a mapping/transfer function to changes in electrode position, effectively determining a robustness of the mapping/transfer function.

In particular, process 600 comprises a step 610 of obtaining the mapping function. The mapping function is able to determine/predict, based on an obtained electrical response of an electrode to the crossing electrode fields, the predicted position of the electrode. This mapping function may, for instance, be obtained from the mapping function generator.

The process 600 also comprises a step 620 of determining a sensitivity of the mapping function to changes in the obtained electrical responses, to thereby generate a quality measure. This measure of sensitivity may represent a quality of the mapping function.

In particular, high spatial gradients or excessive nonlinearities in the mapping function make the mapping more sensitive to errors; meaning that deviation from global scaling factors could therefore represent a bigger error risk. In other words, the greater the sensitivity of the mapping function, the greater the risk of error in the positions predicted by the mapping function, and the lower the quality of the mapping function.

It will be appreciated that this measure of sensitivity can indicate a sensitivity of the mapping function at different locations/areas of the investigated part of the anatomical structure. Thus, the sensitivity may be a local sensitivity or a global sensitivity.

Step 620 may comprise, as illustrated, a step 621 of processing sample electrical responses using the mapping function to determine first sample predicted positions of the electrodes. The sample electrical responses may be real-life electrical responses (e.g. obtained from the electrode(s) mounted on the interventional device) and/or synthetic/imitation electrical responses (e.g. generated by a pseudo-random generator or responsive to a user input).

Preferably, the sample electrical responses are electrical responses that are expected (or known) to map to (predicted) positions in a same area/part of the investigated part of the anatomical structure. This facilitates identification of localized sensitivity of the mapping function (i.e. a sensitivity at a particular part of the investigated part of the anatomical structure).

For determining a local sensitivity, only sample electrical responses representing positions within a particular part of the investigated anatomical structure are obtained. For determining a global sensitivity, a range of sample electrical responses across different positions of the anatomical structure can be used.

Step 620 may further comprise a step 622 of modifying the sample electrical responses, e.g. by applying noise or pseudo-noise to the sample electrical responses. Thus, each sample electrical response corresponds to one or more modified sample electrical response.

Step 620 then moves to a step 623 of processing the modified sample electrical responses using the mapping function to determine second sample predicted positions of the electrodes. It will be clear that each first sample predicted position corresponds to a respective second sample predicted position (which is calculated using the modified version of the electrical response used to generate the first sample predicted position).

Step 620 then moves to a step 624 of determining a sensitivity of the mapping function based on the first sample predicted positions and the second sample predicted positions. For instance, step 620 may comprise determining an (average) distance between the first and second sample predicted position(s).

The value of the sensitivity can be used to determine the quality measure, e.g. making the value of the sensitivity the quality measure itself or by comparing the value of the sensitivity to some predetermined value to generate the quality measure.

Turning back to Figure 5, step 520 of method 500 comprises controlling a user interface to provide a user-perceptible representation of the at least one quality measure. This may comprise controlling a user interface to display a visual representation of the at least one quality measure. The user interface may comprise a display, a screen, a monitor, a speaker, a buzzer, a haptic output, a vibrator and so on.

The representation of different (types of) quality measures may be output using different approaches, e.g. via different types of visual representation or visual output, using different sounds or different vibrations.

In particularly preferable examples, visual representations of the quality measure(s) are displayed with respect to a visual representation of the anatomical model. In other words, step 520 may comprise adjusting or controlling a user interface displaying a visual representation of the anatomical model generated using the dielectric imaging process.

It will be appreciated that different parts of the anatomical cavity are represented by different parts of the anatomical model. A quality measure that indicates a quality of the mapping function with respect to a particular part of the anatomical cavity may be indicated by reference to the corresponding part of the visual representation of the anatomical model. Moreover, a quality of a mapping function represents a quality of the anatomical model.

In some embodiments, for local quality measures, the position of the visual representation of the quality measure is responsive to the position of the area of the anatomical model that represents a part of the anatomical cavity represented by the local quality measure.

In one example, a first type of quality measure (e.g. an accuracy) is communicated by adjusting a color of the (part of the) anatomical model responsive to the quality measure. In another example, a second type of quality measure (e.g. a confidence/sensitivity) is communicated by adjusting a transparency of the (part of the) anatomical model.

Using this approach, a clinician can be made simultaneously aware both of the extent of the local unreliableness and which areas might improve by extra probing with the interventional device.

Other mechanisms for indicating the different quality measures are envisaged, e.g. the use of multiple colors, the use of greyscale/shading, the use of a changing intensity over time for inaccurate areas.

In particularly preferable examples, the user interface may be further configured to provide a visual representation of the (current position of the) interventional device with respect to the anatomical model. It has previously been explained how the position of the interventional device with respect to the anatomical model can be tracked even after the anatomical model is generated, by monitoring the electrical response of the electrode(s) mounted on the interventional device and using the transfer function to determine a position in Euclidian/Cartesian co-ordinate space (relative to the anatomical model in the same space).

Step 520 may comprise modifying the visual representation of the interventional device responsive to the quality measure(s). For instance, a color of the tip of the interventional device may be changed responsive to a local quality measure representing an area in the vicinity of the position of the tip of the interventional device.

This approach provides an improved mechanism for communicating quality information to a user, as it facilitates user control over the quality measure information indicated using a visual representation, thereby providing an improved human-user interface. In particular, feedback as to a local quality of the anatomical model can be dynamically provided to a user by tracking the current (i.e. ongoing) position of the interventional device.

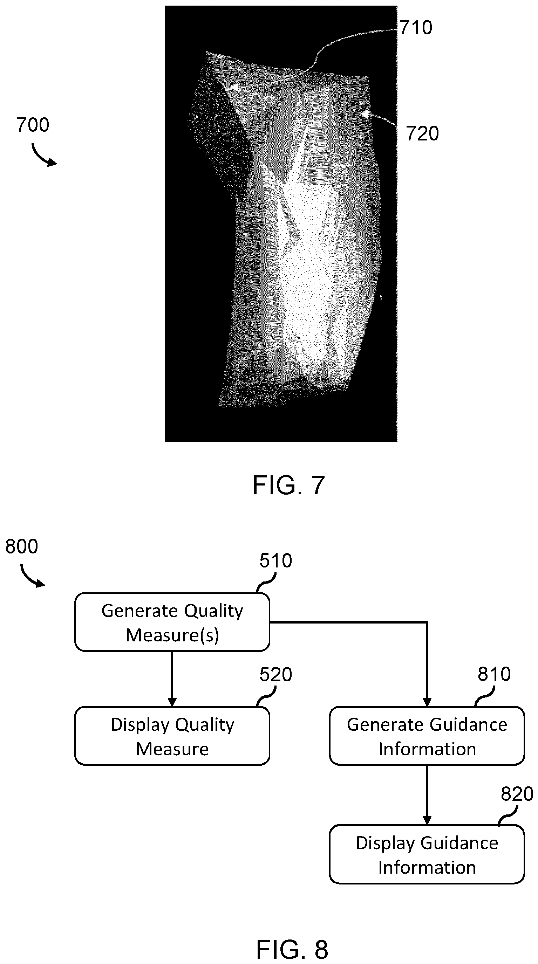

Figure 7 illustrates one example of a display 700 in which quality measures are displayed with respect to a visual representation of an anatomical model. In particular, local quality measures are used to color or shade (e.g. change a saturation level of) different parts of the anatomical model responsive to the value of the local quality measure that represents the part.

As an example, a first level of shading 710 may indicate an area of the anatomical model that represents a part of the anatomical structure for which the mapping function has a low quality, as indicated by a local quality measure for that part of the anatomical model. This may indicate to a user that additional data needs to be acquired in the area of the anatomical cavity represented by that part of the anatomical model.

As another example, a second level of shading 720 may indicate an area of high quality, as indicated by the local quality measure for that part of the anatomical model. This may indicate to a user that no additional data is required at that area of the anatomical cavity represented by that part of the anatomical model.

It will be appreciated that different types of quality measure may be represented using different types of display. For instance, the shading (e.g. saturation) of Figure 7 may indicate an accuracy measure, whereas other forms of visual indicator (e.g. patterns, color, transparency level and so on) may indicate other forms of quality measure.

Figure 8 illustrates a method 800 according to a further embodiment of the invention.

In this embodiment, the step 510 of generating a quality measure comprises generating one or more local quality measures, each local quality measure representing a predicted quality of the mapping function for only a part of the (known) anatomical cavity.

The method 800 further comprises a step 810 of generating guidance information for guiding a user to reposition the interventional device at a location of the anatomical cavity based on the determined one or more local quality measures.

The guidance information may, for instance, be information for guiding a user to reposition the interventional device within an area of the anatomical cavity for which the corresponding quality measure is below some predetermined threshold. This information may, for example, comprise a path for guiding the user to move the interventional device towards a desired location (e.g. a location having a low local quality measure or a location which is predicted to yield the maximum increase of quality for the mapping function, e.g. to meet some desired statistical properties).

In other examples, the guidance information may guide a user to reorient the interventional device, e.g. to obtain additional information for improving the mapping algorithm to correct one or more errors.

Mechanisms for guiding a user to a particular part of the anatomical structure are well known to the skilled person.

In some examples, a (machine-learning) algorithm could be used to process a current location of the interventional device, a desired location for the interventional device and the anatomical model (representing the anatomical structure) to determine a path for moving the interventional device through the anatomical structure to the desired location. This path may form guidance information for display to a user.

As another example, a (machine-learning) algorithm could be used to determine a path between different parts of the anatomical cavity associated with quality measures below some predetermined threshold.

In other examples, step 810 is carried by identifying, based on two or more local quality measures, a part of the anatomical cavity associated with the lowest quality. The guidance information can then be designed for guiding a user to reposition the interventional device based on the identified part of the anatomical cavity associated with the lowest quality.

The guidance information may be output to a user in the form of a user-perceptible output, e.g. in a step 820. Various mechanisms for providing this user-perceptible output are envisaged including audio output (e.g. providing audio instructions on how to move the interventional device) or visual output (e.g. highlighting area or regions of the anatomical model representing an area to which the interventional device should be moved; providing written instructions; or providing annotations such as arrows for guiding the movement of an interventional device).

Using the proposed approaches facilitates a highly efficient roving strategy for the interventional device to be performed.

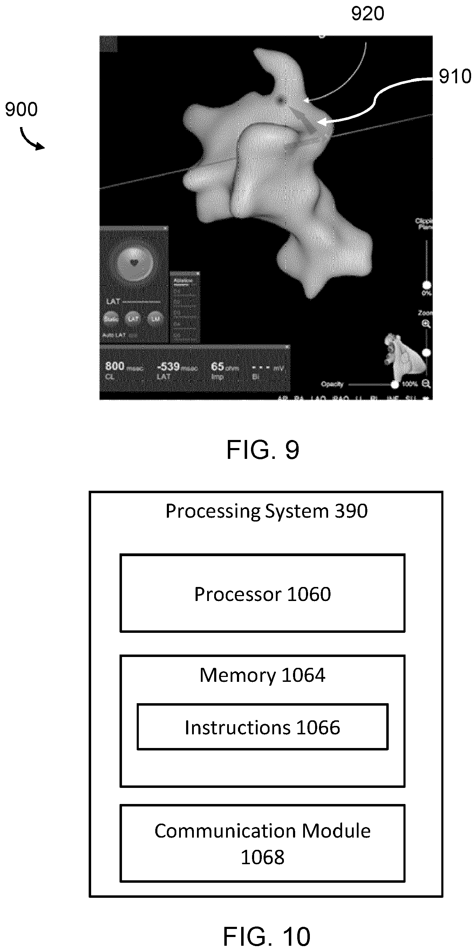

Figure 9 illustrates an example display 900 of guidance information.

The display 900 provides a visual representation of an anatomical model, which is generated and displayed according to previously explained examples. The guidance information indicates a direction (e.g. using an arrow 910) in which an interventional device is to be moved to arrive at a desired location (also indicated using a dot 920 or highlight). The arrow 910 and dot 920 thereby represents a visual representation of guidance information.

Figure 10 is a schematic diagram of a processing system 390, according to embodiments of the present disclosure. As shown, the processing system 390 may include a (data) processor 1060, a memory 1064, and a communication module 1068. These elements may be in direct or indirect communication with each other, for example via one or more buses.

The processor 1060 may include a central processing unit (CPU), a digital signal processor (DSP), an ASIC, a controller, an FPGA, another hardware device, a firmware device, or any combination thereof configured to perform the operations described herein. The processor 1060 may also be implemented as a combination of computing devices, e.g., a combination of a DSP and a microprocessor, a plurality of microprocessors, one or more microprocessors in conjunction with a DSP core, or any other such configuration. In some embodiments, the processor is a distributed processing system, e.g. formed of a set of distributed processors.

The memory 1064 may include a cache memory (e.g., a cache memory of the processor 1060), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 1064 includes a non-transitory computer-readable medium. The non-transitory computer-readable medium may store instructions. For example, the memory 1064, or non-transitory computer-readable medium may have program code recorded thereon, the program code including instructions for causing the processing system 390, or one or more components of the processing system 390, particularly the processor 1060, to perform the operations described herein. For example, the processing system 390 can execute operations of the method 700. Instructions 1066 may also be referred to as code or program code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. The memory 1064, with the code recorded thereon, may be referred to as a computer program product.

The communication module 1068 can include any electronic circuitry and/or logic circuitry to facilitate direct or indirect communication of data between the processing system 390, the penetration device and/or the user interface (or other further device). In that regard, the communication module 1068 can be an input/output (I/O) device. In some instances, the communication module 1068 facilitates direct or indirect communication between various elements of the processing system 390 and/or the system (Figure 2).

It will be understood that disclosed methods are preferably computer-implemented methods. As such, there is also proposed the concept of a computer program comprising computer program code for implementing any described method when said program is run on a processing system, such as a computer or a set of distributed processors.

Different portions, lines or blocks of code of a computer program according to an embodiment may be executed by a processing system or computer to perform any herein described method. In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

The present disclosure proposes a computer program (product) comprising instructions which, when the program is executed by a computer or processing system, cause the computer or processing system to carry out (the steps of) any herein described method. The computer program (product) may be stored on a non-transitory computer readable medium.

Similarly, there is also proposed a computer-readable (storage) medium comprising instructions which, when executed by a computer or processing system, cause the computer or processing system to carry out (the steps of) any herein described method. There is also proposed computer-readable data carrier having stored thereon the computer program (product) previously described. There is also proposed a data carrier signal carrying the computer program (product) previously described.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A processing system (390) for providing a quality measure of a mapping function, wherein the mapping function predicts a position of an electrode (331, 332, 333), within an anatomical cavity (395), using the electrical response of the electrode to crossing electric fields induced within the anatomical cavity, wherein the processing system is configured to:
generate (510, 600) at least one quality measure, each quality measure representing a quality of the electrode position predicting performed by the mapping function with respect to a particular portion of the anatomical cavity; and
control (520) a user interface to provide a user-perceptible representation (710, 720) of the at least one quality measure

2. The processing system (390) of claim 1, wherein the processing system is configured to generate at least one quality measure by:
obtaining (511), from a processor that generates the mapping function, the predicted position of two or more electrodes, mounted on an interventional device, within the anatomical cavity for different positions and/or orientations of the interventional device within the anatomical cavity;
determining (512) one or more characteristics of the predicted positions of the two or more electrodes based on the determined predicted positions; and
determining (513), based on the one or more characteristics, the quality measure.

3. The processing system (390) of claim 2, wherein the one or more characteristics comprise one or more statistical measures of the predicted positions of the two or more electrodes.

4. The processing system (390) of claim 2 or 3, wherein the one or more characteristics comprises a density of the predicted positions and/or a uniformity of the distribution of the predicted positions.

5. The processing system (390) of claims 1 to 4, wherein the processing system is configured to generate at least one quality measure by:
obtaining (511), from a processor generating the mapping function, the predicted position of two or more electrodes, mounted on an interventional device, within the anatomical cavity for different positions and/or orientations of the interventional device within the anatomical cavity;
determining (512) one or more characteristics of the different positions and/or orientations of the interventional device based on the determined predicted positions of the two or more electrodes; and
determining (513), based on the one or more characteristics, the quality measure.

6. The processing system (390) of claim 5, wherein:
the step (512) of determining one or more characteristics of the different positions and/or orientations of the interventional device comprises determining, for each position and/or orientation of the interventional device at which electric responses are obtained, an orientation of the interventional device with respect to the anatomical cavity based on the predicted positions for the two or more electrodes at the position and/or orientation of the interventional device; and
the one or more characteristics comprises a uniformity of the distribution of the determined orientations of the interventional device.

7. The processing system (390) of any of claims 1 to 6, wherein the processing system is configured to generate (600) at least one quality measure by determining (620) a sensitivity of the mapping function to changes in the obtained electrical responses.

8. The processing system (390) of claim 7, wherein the processing system is configured to determine a sensitivity of the mapping function by:
processing (621) sample electrical responses using the mapping function to determine first sample predicted positions of the electrodes;
modifying (622) the sample electrical responses;
processing (623) the modified sample electrical responses using the mapping function to determine second sample predicted positions of the electrodes; and
determining (624) a sensitivity of the mapping function based on the first sample predicted positions and the second sample predicted positions.

9. The processing system (390) of any of claims 1 to 8, wherein the processing system is configured to generate at least one quality measure by determining an accuracy of obtained electrical responses of the two or more electrodes.

10. The processing system (390) of any of claims 1 to 9, wherein the step (510, 600) of determining a quality measure comprises determining a global quality measure representing the predicted overall quality of the mapping function for predicting the position of an electrode within the entire known anatomical cavity.

11. The processing system (390) of any of claims 1 to 10, wherein the step (510, 600) of determining a quality measure comprises determining one or more local quality measures, each local quality measure representing a predicted quality of the mapping function for predicting a position of an electrode within only a part of the anatomical cavity.

12. The processing system (390) of any claim 11, wherein the processing system is further configured to generate (810) guidance information for guiding a user to reposition the interventional device at a location of the anatomical structure based on the determined one or more local quality measures.

13. The processing system (390) of claim 12, wherein:
the processing system is configured to identify, based on the determined one or more local quality measures, a part of the anatomical cavity associated with the lowest quality; and
the guidance information is designed for guiding a user to reposition the interventional device based on the identified part of the anatomical cavity associated with the lowest quality.

14. A computer-implemented method (500, 800) for providing a quality measure of a mapping function, wherein the mapping function predicts a position of an electrode (331, 332, 333), within an anatomical cavity (395), using the electrical response of the electrode to crossing electric fields induced within the anatomical cavity, the computer-implemented method comprising:
generating (510, 600) at least one quality measure, each quality measure representing a quality of the electrode position predicting performed by the mapping function with respect to a particular portion of the anatomical cavity; and
controlling (520) a user interface to provide a user-perceptible representation of the at least one quality measure.

15. A computer program product comprising computer program code means which, when executed on a computing device having a processing system, cause the processing system to perform all of the steps of the method according to claim 14.
